# EUROPEAN PATENT SPECIFICATION

(11) **EP 3 952 993 B1**
(45) Date of publication and mention of the grant of the patent: **08.01.2025**
(21) Application number: 20710208.8
(22) Date of filing: 17.03.2020
(51) Int. Cl.: A61P 17/06, C07K 16/24

(54) **METHOD OF TREATING FUMARIC ACID ESTER-RESISTANT PLAQUE PSORIASIS**
VERFAHREN ZUR BEHANDLUNG VON FUMARSÄUREESTER-RESISTENTER PLAQUE-PSORIASIS
PROCÉDÉ DE TRAITEMENT DU PSORIASIS EN PLAQUES RÉSISTANT AUX ESTERS D'ACIDE FUMARIQUE

(30) Priority: 12.04.2019 EP 19382278
(43) Date of publication of application: 16.02.2022
(73) Proprietor: Almirall S.A., 08022 Barcelona (ES)
(72) Inventor: PAU CHARLES, Ignasi, 08022 Barcelona (ES)
(74) Representative: J A Kemp LLP
(86) International application number: PCT/EP2020/057342
(87) International publication number: WO 2020/207724

(56) References cited:
- NAST A. ET AL: "European S3-Guidelines on the systemic treatment of psoriasis vulgaris - Update 2015 - Short version - EDF in cooperation with EADV and IPC", JEADV , THE JOURNAL OF THE EUROPEAN ACADEMY OF DERMATOLOGY AND VENEREOLOGY, vol. 29, no. 12, 1 December 2015 (2015-12-01), NL, pages 2277 - 2294, XP093066207, ISSN: 0926-9959, Retrieved from the Internet <URL:https://api.wiley.com/onlinelibrary/tdm/v1/articles/10.1111%2Fjdv.13354> DOI: 10.1111/jdv.13354
- POULIN Y. ET AL: "Efficacy of tildrakizumab by patient demographic and disease characteristics across a phase 2b and 2 phase 3 trials in patients with moderate-to-severe chronic plaque psoriasis", JEADV , THE JOURNAL OF THE EUROPEAN ACADEMY OF DERMATOLOGY AND VENEREOLOGY, vol. 34, no. 7, 1 July 2020 (2020-07-01), NL, pages 1500 - 1509, XP093066258, ISSN: 0926-9959, Retrieved from the Internet <URL:https://onlinelibrary.wiley.com/doi/full-xml/10.1111/jdv.16187> DOI: 10.1111/jdv.16187
- FRAMPTON JAMES E: "Tildrakizumab: A Review in Moderate-to-Severe Plaque Psoriasis", AMERICAN JOURNAL OF CLINICAL DERMATOLOGYNEW ZEALANDAPR 2017, ADIS INTERNATIONAL LTD, NZ, vol. 20, no. 2, 31 March 2019 (2019-03-31), pages 295 - 306, XP009516110, ISSN: 1179-1888, DOI: 10.1007/S40257-019-00435-9
- TODD WECHTER ET AL: "Targeting p19 as a treatment option for psoriasis: an evidence-based review of guselkumab", THERAPEUTICS AND CLINICAL RISK MANAGEMENT, vol. Volume 14, 1 January 2018 (2018-01-01), NZ, pages 1489 - 1497, XP055623101, ISSN: 1176-6336, DOI: 10.2147/TCRM.S177127
- M. STICHERLING ET AL: "Secukinumab is superior to fumaric acid esters in treating patients with moderate-to-severe plaque psoriasis who are naive to systemic treatments: results from the randomized controlled PRIME trial", BRITISH JOURNAL OF DERMATOLOGY, vol. 177, no. 4, 1 October 2017 (2017-10-01), UK, pages 1024 - 1032, XP055623176, ISSN: 0007-0963, DOI: 10.1111/bjd.15707

## Description

### FIELD OF THE INVENTION

The present invention relates to a method of treating fumaric acid ester (FAE)-resistant plaque psoriasis, such as dimethyl fumarate (DMF)-resistant plaque psoriasis, in a patient. Specifically, the method comprises administering a therapeutically-effective amount of an anti-IL23 antibody as herein defined, such as tildrakizumab, to said patient.

### BACKGROUND TO THE INVENTION

Psoriasis is a chronic relapsing/remitting, inflammatory, immune-mediated systemic skin disease. Plaque psoriasis (psoriasis vulgaris) is the most common type of psoriasis, characterized by sharply demarcated erythematous and scaly lesions/plaques with a silvery appearance (see Feldman SR et al., Am Health Drug Benefits, December 2016;9(9):504-13 and Pathirana D et al., J Eur Acad Dermatol Venereol. October 2009;23:1-70). In Western industrialized countries plaque psoriasis has a prevalence of around 2% (Pathirana D et al., J Eur Acad Dermatol Venereol. October 2009;23:1-70). Psoriasis is associated with an increased risk of developing serious clinical conditions such as inflammatory arthritis (psoriatic arthritis), and cardiovascular and other non-communicable diseases. Likewise, psoriasis causes great physical, emotional and social burden that lead to impairment in health-related quality of life (HRQoL) of people with psoriasis (see Pathirana D et al., J Eur Acad Dermatol Venereol. October 2009;23:1-70 and Menter A et al., J Am Acad Dermatol. May 2008;58(5):826-50).

Systemic therapy of psoriasis with fumarates, also known as fumaric acid esters (FAEs), was first investigated by a German scientist with psoriasis who experimented on himself and published his results in 1959. However, it was not until 1994 that the first fumarate-based drug was approved in Germany for the treatment of severe psoriasis. This was marketed under the name of Fumaderm^{®}, and represents a combination of DMF and three salts of monoethyl fumarate (see Mrowietz U et al., J Eur Acad Dermatol Venereol. October 2018;32:3-14 and Nast A et al., Arch Dermatol Res. June 2007;299(3):111-38).

DMF is considered the active component in the combination, as the monoethyl fumarate salts alone have not been shown to have significant clinical efficacy (Landeck L et al., Arch Dermatol Res. August 2018; 310(6):475-83). The initial indication of Fumaderm^{®} was expanded to include moderate disease in 2008 (Mrowietz U et al., J Eur Acad Dermatol Venereol. October 2018;32:3-14). The use of FAEs in most of the European Union (EU) remained unlicensed until 2017 when the EMA approved Skilarence^{®}, an oral formulation of DMF, for the treatment of adults with moderate-to-severe chronic plaque psoriasis in need of systemic medical therapy (see Mrowietz U et al., J Eur Acad Dermatol Venereol. October 2018;32:3-14 and the summary of product characteristics on Skilarence 30 mg gastro-resistant tablets, available from the European Medicines Agency (EMA)).

DMF is considered as a pro-drug for oral use to generate sufficient blood and tissue levels of monomethyl fumarate (MMF), the active in vivo metabolite. The immunomodulating and anti-inflammatory effects of MMF have not been clearly established (see Mrowietz U et al., J Eur Acad Dermatol Venereol. October 2018;32:3-14). However, without wishing to be bound by theory, it is thought that MMF effects are mainly due to the interaction with the intracellular reduced glutathione of cells directly involved in the pathogenesis of psoriasis. This interaction with glutathione leads to the inhibition of translocation into the nucleus and the transcriptional activity of the nuclear factor kappa-light-chain-enhancer of activated B-cells (NF-κB). The main activity of DMF and MMF is considered to be immunomodulatory, resulting in a shift in T helper (Th) cells from the Th1 and Th17 profile to a Th2 phenotype. The inflammatory cytokine production is reduced with induction of pro-apoptotic events, inhibition of keratinocyte proliferation, reduced expression of adhesion molecules, and diminished inflammatory infiltrate within psoriatic plaques (see the summary of product characteristics on Skilarence 30 mg gastro-resistant tablets, available from the European Medicines Agency (EMA)).

In a double-blind, placebo-controlled, non-inferiority phase III trial, the efficacy and safety of DMF (up to 720 mg/daily) was compared to Fumaderm^{®} and placebo (Mrowietz U et al., Br J Dermatol 2016). A total of 671 patients diagnosed with moderate-to-severe psoriasis were randomized (2:2:1, respectively) to be treated for 16 weeks. The proportion of patients achieving an improvement (reduction) of ≥ 75% in Psoriasis Area and Severity Index (PASI) score compared to the score at the visit of the first drug administration (PASI 75) and the proportion achieving clear/almost clear in the Physician's Global Assessment (PGA) were used as co-primary endpoints. DMF was shown to be significantly superior to placebo and non-inferior to FAEs in both endpoints. The safety profile of DMF was also similar to FAEs, being gastrointestinal disorders the most frequently drug-related adverse events (AEs). No unexpected safety issues were detected. Based on this trial, DMF was approved by the EMA in June 2017 to treat moderate-to-severe plaque psoriasis in patients in need of systemic drug treatment (see the summary of product characteristics on Skilarence 30 mg gastro-resistant tablets, available from the European Medicines Agency (EMA)).

However, despite the above, it has been recognised that there are a number of instances of FAE-resistant plaque psoriasis amongst the patients who are treated with these compounds, including many instances of DMF-resistant plaque psoriasis. Accordingly, there is a need for alternative therapeutic approaches for treating FAE-resistant plaque psoriasis.

### SUMMARY OF THE INVENTION

It has been surprisingly discovered that anti-IL23 antibodies as herein defined, and in particular tildrakizumab, may be used to treat FAE-resistant plaque psoriasis. In particular, the present inventors have noted, based on unpublished results from the clinical trial described in Example 1, that tildrakizumab is effective in treating FAE-resistant plaque psoriasis. Specifically, unpublished data from the above clinical trial indicates that some patients who were successfully treated with tildrakizumab during the clinical trials were suffering from FAE-resistant plaque psoriasis (i.e. had previously received an unsuccessful treatment with DMF). Any reference to a method of treatment practised on the human or animal body is to be interpreted as substances and compositions for use in such treatment.

The present invention therefore provides a method of treating FAE-resistant plaque psoriasis in a patient, said method comprising administering a therapeutically-effective amount of an anti-IL23 antibody to said patient, wherein said anti-IL23 antibody comprises a set of six CDRs, HCDR1, HCDR2, HCDR3, LCDR1, LCDR2 and LCDR3, wherein:
(a) HCDR1, HCDR2 and HCDR3 are contained within a heavy chain variable region (HCVR) having an amino acid sequence of SEQ ID No. 1, and LCDR1, LCDR2 and LCDR3 are contained within a light chain variable region (LCVR) having an amino acid sequence of SEQ ID No. 2; or
(b) HCDR1 has the amino acid sequence of SEQ ID NO: 5; HCDR2 has the amino acid sequence of SEQ ID NO: 6; HCDR3 has the amino acid sequence of SEQ ID NO: 7; LCDR1 has the amino acid sequence of SEQ ID NO: 8; LCDR2 has the amino acid of SEQ ID NO: 9; and LCDR3 has the amino acid sequence of SEQ ID NO: 10.

The present invention further provides an anti-IL23 antibody for use in a method of treating of fumaric acid ester-resistant plaque psoriasis in a patient, wherein said method is as defined above.

### DETAILED DESCRIPTION OF THE INVENTION

The terms "treatment" and "treating" as used herein refers to the treatment of a disease or medical condition in a human patient which includes:
(a) preventing the disease or medical condition from occurring, i.e., prophylactic treatment of a patient;
(b) ameliorating the disease or medical condition, i.e., causing regression of the disease or medical condition in a patient;
(c) suppressing the disease or medical condition, i.e., slowing the development of the disease or medical condition in a patient; or
(d) alleviating the symptoms of the disease or medical condition in a patient.

The term "therapeutically effective amount" of a compound or composition as used herein means an amount sufficient to cure, alleviate or partially arrest the clinical manifestations of a given disease and its complications. An amount adequate to accomplish this is defined as "therapeutically effective amount". Effective amounts for each purpose will depend on the severity of the disease or injury as well as the weight and general state of the subject. It will be understood that determining an appropriate dosage may be achieved using routine experimentation, by constructing a matrix of values and testing different points in the matrix, which is all within the ordinary skills of a trained physician.

### FAE-resistant plaque psoriasis and the patient having FAE-resistant plaque psoriasis

The plaque psoriasis to be treated in the present invention is a FAE-resistant plaque psoriasis (i.e. plaque psoriasis which is resistant or refractory to treatment with FAEs).

Typically, the FAE to which the plaque psoriasis is resistant is dimethyl fumarate (DMF). That is to say, typically the plaque psoriasis to be treated in the present invention is DMF-resistant plaque psoriasis.

The plaque psoriasis is preferably a moderate to severe plaque psoriasis. Thus, in one aspect, the plaque psoriasis is a moderate plaque psoriasis. In another aspect, the plaque psoriasis is a severe plaque psoriasis. The diagnosis and severity of plaque psoriasis can easily be made by a skilled person using standard test procedures. Suitable test procedures include the Psoriasis Area Severity Index (PASI), the Body Surface Area (BSA) assessment, the Physician's Global Assessments (PGA) - including the palmoplantar PGA (PPPGA) and the scalp PGA (ScPGA), the Pruritus - Visual Analogue Scale (Pruritus-VAS), the Medical Outcome Sleep Study (MOSS), the Dermatology Life Quality Index (DLQI), and the Skindex-16. Details on each of these procedures are provided below. PASI is preferred in the present invention for the diagnosing and assessing the severity of plaque psoriasis. Typically, the plaque psoriasis is moderate to severe plaque psoriasis if the patient exhibits a PASI score of ≥ 10.

The plaque psoriasis may be present on any part of the body. Thus, it may be present on one or more of the head (including on the scalp) and neck, the upper extremities, the trunk, and the lower extremities.

The patient for treatment in the present invention is a patient having FAE-resistant plaque psoriasis. That is to say, the patient suffers from plaque psoriasis, and that plaque psoriasis is refractory or resistant to treatment with FAEs. Thus, the patient may also be described as one who is suffering from plaque psoriasis and is unresponsive (or does not respond) to treatment with FAEs. Such patients are known as "non-responder" patients. By "unresponsive" it is meant that the plaque psoriasis is not successfully treated by the FAE (i.e. the plaque psoriasis is resistant to treatment with the FAE). For the avoidance of doubt, the FAE may nevertheless cause a response not linked to treatment of plaque psoriasis in the patient. The present invention thus also provides a method of treating plaque psoriasis in a patient who is unresponsive (or does not respond) to treatment with FAEs, said method comprising administering a therapeutically-effective amount of an anti-IL23 antibody as defined herein to said patient.

The patient may be any subject having a FAE-resistant plaque psoriasis. However, typically, the patient is a human. Typically, the patient is an adult. Thus, the patient is preferably a human adult.

The patient may be afflicted with other forms of psoriasis in addition to plaque psoriasis, such as Guttate psoriasis, inverse psoriasis, pustular psoriasis, erythrodermic psoriasis and/or psoriatic arthritis. Alternatively, the patient may be suffering only from plaque psoriasis and not be suffering from other forms of psoriasis, such as those listed above.

A skilled person can easily determine whether plaque psoriasis is FAE-resistant, as discussed in further detail below. Patients who are unresponsive (or do not respond) to treatment with FAEs (i.e. "non-responder" patients) can be identified in the same manner.

Typically, FAE-resistant plaque psoriasis is a plaque psoriasis assessed as being resistant to treatment with a FAE, such as DMF, following a treatment regime with the treatment lasting for at least 4 weeks, preferably at least 6 weeks, more preferably at least 9 weeks, more preferably still at least 12 weeks, and most preferably at least 16 weeks, for example about 16 weeks. Preferably, the FAE-resistant plaque psoriasis is a plaque psoriasis assessed as being resistant to treatment with a FAE, such as DMF, following a treatment regime with the FAE DMF lasting for 16 weeks or about 16 weeks.

The treatment regime with a FAE, such as DMF, will typically be carried out according to a standard regime known to those skilled in the art (for example as defined in the summary of product characteristics on Skilarence 30 mg gastro-resistant tablets, available from the European Medicines Agency (EMA)).

The "standard regime" with DMF typically involves DMF 30 mg once daily in the first week, DMF 30 mg twice daily in the second week, DMF 30 mg three times daily in the third week, DMF 120 mg once daily in the fourth week, DMF 120 mg twice daily in the fifth week, DMF 120 mg three times daily in the sixth week, DMF 120 mg four times daily in the seventh week, DMF 120 mg five times daily in the eighth week, and DMF 120 mg six times daily in the ninth and subsequent weeks (e.g. typically up to 16 weeks). A "standard regime" with an FAE other than DMF will typically be similar to that above.

A particularly preferred "standard regime" with DMF is carried out as follows:
In the first week, the patient takes DMF 30 mg once daily (one tablet in the evening). In the second week, the patient takes DMF 30 mg twice daily (one tablet in the morning and one in the evening). In the third week, the patient takes DMF 30 mg three times daily (one tablet in the morning, one at midday, and one in the evening). From the fourth week, treatment is switched to one tablet of DMF 120 mg in the evening. The patient then increases the daily DMF dose by one 120 mg tablet per week for the subsequent 5 weeks. Thus, in the fifth week, the patient takes DMF 120 mg twice daily (one tablet in the morning and one in the evening). In the sixth week, the patient takes DMF 120 mg three times daily (one tablet in the morning, one at midday, and one in the evening). In the seventh week, patient takes DMF 120 mg four times daily (one tablet in the morning, one at midday, and two in the evening). In the eighth week, the patient takes DMF 120 mg five times daily (two tablets in the morning, one at midday, and two in the evening). In the ninth week, the patient takes DMF 120 mg six times daily (two tablets in the morning, two at midday, and two in the evening) - the maximum daily dose. The maximum dosage is maintained for the subsequent weeks.

Preferably, therefore, the FAE-resistant plaque psoriasis is DMF-resistant plaque psoriasis, and the plaque psoriasis is resistant to treatment to a DMF treatment regime lasting for 16 weeks according to the standard regime recited above, which typically involves DMF 30 mg once daily in the first week, DMF 30 mg twice daily in the second week, DMF 30 mg three times daily in the third week, DMF 120 mg once daily in the fourth week, DMF 120 mg twice daily in the fifth week, DMF 120 mg three times daily in the sixth week, DMF 120 mg four times daily in the seventh week, DMF 120 mg five times daily in the eighth week, and DMF 120 mg six times daily in the ninth and subsequent weeks.

The person skilled in the art can readily assess whether a plaque psoriasis is resistant (or refractory) to treatment with a FAE, such as DMF, through assessing the efficacy of the FAE treatment using standard test procedures. As noted above, suitable tests for diagnosing and assessing the severity of plaque psoriasis include the Psoriasis Area Severity Index (PASI), the Body Surface Area (BSA) assessment, the Physician's Global Assessments (PGA) - including the palmoplantar PGA (PPPGA) and the scalp PGA (ScPGA), the Pruritus - Visual Analogue Scale (Pruritus-VAS), the Medical Outcome Sleep Study (MOSS), the Dermatology Life Quality Index (DLQI), and the Skindex-16. Details on each of these procedures are given in turn below. However, PASI is preferred for assessing if a plaque psoriasis is FAE-resistant.

### Psoriasis Area Severity Index (PASI)

The PASI is a scoring method that can be used for the diagnosis, assessment and grading of the severity of psoriasis patients. The severity of the disease is calculated by scoring the signs of the disease (erythema, induration and scaling) for each body region. Overall scores range from 0 (no psoriasis) to 72 (the most severe disease).

A PASI 75 response, is defined as having an improvement (reduction) of ≥ 75% in PASI scores compared to the baseline score. A PASI 50 response, is defined as having an improvement (reduction) of ≥ 50% in PASI scores compared to the baseline score.

Typically, according to the present invention, plaque psoriasis is resistant to treatment with a FAE (such as DMF) if the patient does not exhibit a response of PASI 50 (i.e. does not exhibit an improvement (reduction) of ≥50% in PASI score compared to the baseline score) following a treatment regime with FAEs (i.e. typically a treatment regime as defined above). Thus, the plaque psoriasis is FAE-resistant if the patient exhibits a reduction of <50% in PASI score compared to the baseline score following a treatment regime with the FAE. Preferably, the reduction in PASI score in FAE-resistant plaque psoriasis is <40% compared to the baseline score, more preferably <30%, more preferably <20%, most preferably <10% or about 0%. For the avoidance of doubt, some patients may exhibit an increase in PASI score following a treatment regime with the FAE, such as DMF.

Most preferably, therefore, the FAE-resistant plaque psoriasis is DMF-resistant plaque psoriasis, in which the patient exhibits a reduction of <50% in PASI score compared to the baseline score following a DMF treatment regime lasting for 16 weeks and involving DMF 30 mg once daily in the first week, DMF 30 mg twice daily in the second week, DMF 30 mg three times daily in the third week, DMF 120 mg once daily in the fourth week, DMF 120 mg twice daily in the fifth week, DMF 120 mg three times daily in the sixth week, DMF 120 mg four times daily in the seventh week, DMF 120 mg five times daily in the eighth week, and DMF 120 mg six times daily in the ninth and subsequent weeks.

### Body Surface Area (BSA)

The BSA assessment measures the total area of the body affected by psoriasis. Using the surface of the hand to equal 1% BSA, a physician may assess the total BSA affected. It is the patient's palm, including the five digits, that is used as the reference (to represent approximately 1% of the total body surface area) and is used to repeatedly cover the lesions on the body. The physician totals the number of palms required and then estimates the percentage in each of the four body regions.

The four body regions are:
- Head (including scalp) and neck (10%)
- Upper extremities (20%)
- Trunk (30%)
- Lower extremities (40%)

Typically, according to the present invention, the plaque psoriasis is FAE-resistant if the patient exhibits a reduction in BSA afflicted with plaque psoriasis of <50% compared to the baseline BSA afflicted with plaque psoriasis following a treatment regime with the FAE (i.e. typically a treatment regime as defined above). Preferably, the reduction in BSA afflicted with plaque psoriasis in FAE-resistant plaque psoriasis is <40% compared to the baseline BSA afflicted with plaque psoriasis, more preferably <30%, more preferably <20%, most preferably <10% or about 0%. For the avoidance of doubt, some patients may exhibit an increase in the baseline BSA afflicted with plaque psoriasis following a treatment regime with the FAE, such as DMF.

Most preferably, therefore, the FAE-resistant plaque psoriasis is DMF-resistant plaque psoriasis, in which the patient exhibits a reduction in the baseline BSA afflicted with plaque psoriasis of <50% compared to the baseline BSA afflicted with plaque psoriasis following a DMF treatment regime lasting for 16 weeks and involving DMF 30 mg once daily in the first week, DMF 30 mg twice daily in the second week, DMF 30 mg three times daily in the third week, DMF 120 mg once daily in the fourth week, DMF 120 mg twice daily in the fifth week, DMF 120 mg three times daily in the sixth week, DMF 120 mg four times daily in the seventh week, DMF 120 mg five times daily in the eighth week, and DMF 120 mg six times daily in the ninth and subsequent weeks.

### Physician's Global Assessments (PGA) - including the palmoplantar PGA (PPPGA) and the scalp PGA (ScPGA)

The PGA is an assessment by a physician of the overall disease severity at the time of evaluation. The PGA is a 6-point scale ranging from 0 (clear) to 5 (severe), incorporating an assessment of the severity of the three primary signs of the disease: erythema, scaling and plaque elevation. When making the assessment of overall severity, the physician should factor in areas that have already been cleared (i.e., have scores of 0) and not just evaluate remaining lesions for severity, i.e., the severity of each sign is averaged across all areas of involvement, including cleared lesions.

The palmoplantar PGA (PPPGA) is an assessment by the physician of the palmoplantar disease severity at the time of evaluation. The PPPGA is a 5-point scale ranging from 0 (clear) to 4 (severe).

Likewise, the scalp PGA (ScPGA) is an assessment by the physician of the scalp disease severity at the time of evaluation. The ScPGA is also 5-point scale ranging from 0 (clear) to 4 (severe).

Typically, according to the present invention, the plaque psoriasis is FAE-resistant if the patient exhibits maintained or increased PGA, PPPGA and/or ScPGA scores compared to the baseline PGA, PPPGA and/or ScPGA scores following a treatment regime with the FAE (i.e. typically a treatment regime as defined above).

Preferably, therefore, the FAE-resistant plaque psoriasis is DMF-resistant plaque psoriasis, in which the patient exhibits maintained or increased PGA, PPPGA and/or ScPGA scores compared to the baseline PGA, PPPGA and/or ScPGA scores following a DMF treatment regime lasting for 16 weeks and involving DMF 30 mg once daily in the first week, DMF 30 mg twice daily in the second week, DMF 30 mg three times daily in the third week, DMF 120 mg once daily in the fourth week, DMF 120 mg twice daily in the fifth week, DMF 120 mg three times daily in the sixth week, DMF 120 mg four times daily in the seventh week, DMF 120 mg five times daily in the eighth week, and DMF 120 mg six times daily in the ninth and subsequent weeks.

### Pruritus - Visual Analogue Scale (Pruritus- VAS)

The pruritus-VAS is a single-item continuous scale comprised of a 10 cm (100 mm) horizontal/vertical line anchored by two verbal descriptors, one for each symptom extreme. For pruritus intensity, the scale is anchored by "no pruritus" (score of 0) and "worst imaginable pruritus" (score of 100 mm).

Typically, according to the present invention, the plaque psoriasis is FAE-resistant if the patient reports a maintained or increased pruritus-VAS score compared to the baseline pruritus-VAS score following a treatment regime with the FAE (i.e. typically a treatment regime as defined above).

Preferably, therefore, the FAE-resistant plaque psoriasis is DMF-resistant plaque psoriasis, in which the patient reports a maintained or increased pruritus-VAS score compared to the baseline pruritus-VAS score following a DMF treatment regime lasting for 16 weeks and involving DMF 30 mg once daily in the first week, DMF 30 mg twice daily in the second week, DMF 30 mg three times daily in the third week, DMF 120 mg once daily in the fourth week, DMF 120 mg twice daily in the fifth week, DMF 120 mg three times daily in the sixth week, DMF 120 mg four times daily in the seventh week, DMF 120 mg five times daily in the eighth week, and DMF 120 mg six times daily in the ninth and subsequent weeks.

### Medical Outcome Sleep Study (MOSS)

The MOSS questionnaire consists of 12 items leading to 6 subscales or domains: sleep disturbance, sleep adequacy, daytime sleepiness, 'supposed or known' snoring, being awakened by shortness of breath or by a headache, and quantity of sleep. Subscales are standardised to yield scores from zero to 100, with the exception of sleep quantity. Higher scores on the MOSS reflects more of the attribute indicated by the subscale name.

Typically, according to the present invention, the plaque psoriasis is FAE-resistant if the patient reports maintained or increased MOSS scores on the subscales of sleep disturbance, daytime sleepiness, 'supposed or known' snoring, and/or being awakened by shortness of breath or by a headache compared to the baseline MOSS scores following a treatment regime with the FAE (i.e. typically a treatment regime as defined above).

Preferably, therefore, the FAE-resistant plaque psoriasis is DMF-resistant plaque psoriasis, in which the patient reports maintained or increased MOSS scores on the subscales of sleep disturbance, daytime sleepiness, 'supposed or known' snoring, and/or being awakened by shortness of breath or by a headache compared to the baseline MOSS scores following a DMF treatment regime lasting for 16 weeks and involving DMF 30 mg once daily in the first week, DMF 30 mg twice daily in the second week, DMF 30 mg three times daily in the third week, DMF 120 mg once daily in the fourth week, DMF 120 mg twice daily in the fifth week, DMF 120 mg three times daily in the sixth week, DMF 120 mg four times daily in the seventh week, DMF 120 mg five times daily in the eighth week, and DMF 120 mg six times daily in the ninth and subsequent weeks.

Similarly, according to the present invention, the plaque psoriasis is FAE-resistant if the patient reports maintained or decreased MOSS scores on the subscales of sleep adequacy and sleep quantity compared to the baseline MOSS scores following a treatment regime with the FAE (i.e. typically a treatment regime as defined above).

Preferably, therefore, the FAE-resistant plaque psoriasis is DMF-resistant plaque psoriasis, in which the patient reports maintained or decreased MOSS scores on the subscales of sleep adequacy and sleep quantity compared to the baseline MOSS scores following a DMF treatment regime lasting for 16 weeks and involving DMF 30 mg once daily in the first week, DMF 30 mg twice daily in the second week, DMF 30 mg three times daily in the third week, DMF 120 mg once daily in the fourth week, DMF 120 mg twice daily in the fifth week, DMF 120 mg three times daily in the sixth week, DMF 120 mg four times daily in the seventh week, DMF 120 mg five times daily in the eighth week, and DMF 120 mg six times daily in the ninth and subsequent weeks.

### Dermatology Life Quality Index (DLQI)

Patients are asked about the impact of their disease and the respective treatment on their lives. The DLQI score is calculated by summing the score of each question resulting in a maximum of 30 and a minimum of 0. The higher the score, the more quality of life is impaired. The DLQI can also be expressed as a percentage of the maximum possible score of 30.

Typically, according to the present invention, the plaque psoriasis is FAE-resistant if the patient exhibits a maintained or increased DLQI score compared to the baseline DLQI score following a treatment regime with the FAE (i.e. typically a treatment regime as defined above).

Preferably, therefore, the FAE-resistant plaque psoriasis is DMF-resistant plaque psoriasis, in which the patient exhibits a maintained or increased DLQI score compared to the baseline DLQI score following a DMF treatment regime lasting for 16 weeks and involving DMF 30 mg once daily in the first week, DMF 30 mg twice daily in the second week, DMF 30 mg three times daily in the third week, DMF 120 mg once daily in the fourth week, DMF 120 mg twice daily in the fifth week, DMF 120 mg three times daily in the sixth week, DMF 120 mg four times daily in the seventh week, DMF 120 mg five times daily in the eighth week, and DMF 120 mg six times daily in the ninth and subsequent weeks.

### Skindex-16

Skindex-16 is a further dermatological instrument to measure dermatology-specific health-related quality of life (HRQoL). The 16-item Skindex questionnaire is divided into three domains: questions related to the patients' symptoms (1-4), emotions (5-11), and functioning (12-16). Each question asks the patient to quantify how much a specific aspect of their skin condition bothered them in the week prior to administration of the Skindex-16. The questions are answered on a scale from 0 (never bothered) to 6 (always bothered) with a total possible score ranging from 0 (best HRQoL) to 96 (worst HRQoL). Each item is then transformed to a linear scale from 0 to 100.

Typically, according to the present invention, the plaque psoriasis is FAE-resistant if the patient exhibits a maintained or increased Skindex-16 score compared to the baseline Skindex-16 score following a treatment regime with the FAE (i.e. typically a treatment regime as defined above).

Preferably, therefore, the FAE-resistant plaque psoriasis is DMF-resistant plaque psoriasis, in which the patient exhibits a maintained or increased Skindex-16 score compared to the baseline Skindex-16 score following a DMF treatment regime lasting for 16 weeks and involving DMF 30 mg once daily in the first week, DMF 30 mg twice daily in the second week, DMF 30 mg three times daily in the third week, DMF 120 mg once daily in the fourth week, DMF 120 mg twice daily in the fifth week, DMF 120 mg three times daily in the sixth week, DMF 120 mg four times daily in the seventh week, DMF 120 mg five times daily in the eighth week, and DMF 120 mg six times daily in the ninth and subsequent weeks.

### The anti-IL23 antibody

Examples of anti-IL-23 antibodies which may be used in the practice of the present invention, as well as methods for generating the same, are described in WO2008/103432 (e.g. the humanized 13B8-b antibody and the antigen-binding fragments thereof) which is incorporated herein by reference in its entirety, and particularly with respect to the discussion of antibody h13B8b and the antigen binding fragments thereof. Preferably, the antibody for use in the present invention is a humanized antibody.

An antibody or antigen binding fragment thereof for use in the invention may comprise a set of six CDRs, HCDR1, HCDR2, HCDR3, LCDR1, LCDR2 and LCDR3, wherein:
(a) HCDR1, HCDR2 and HCDR3 are contained within a heavy chain variable region (HCVR) having an amino acid sequence of SEQ ID No. 1, and LCDR1, LCDR2 and LCDR3 are contained within a light chain variable region (LCVR) having an amino acid sequence of SEQ ID No. 2; or
(b) HCDR1 has the amino acid sequence of SEQ ID NO: 5; HCDR2 has the amino acid sequence of SEQ ID NO: 6; HCDR3 has the amino acid sequence of SEQ ID NO: 7; LCDR1 has the amino acid sequence of SEQ ID NO: 8; LCDR2 has the amino acid of SEQ ID NO: 9; and LCDR3 has the amino acid sequence of SEQ ID NO: 10.

Methods and techniques for identifying CDRs within HCVR and LCVR sequences will be known to those skilled in the art. Conventions for identifying CDRs include, for example, the Kabat definition, the Chothia definition, and the AbM definition. At a general level, the Kabat definition is based on sequence variability, the Chothia definition is based on the location of the structural loop regions, and the AbM definition is a compromise between the Kabat and Chothia approaches. See, e.g. Kabat, et al., "Sequences of Proteins of Immunological Interest", 5th Ed. Public Health Service, National Institutes of Health, Bethesda, MD. (1991); Al-Lazikani et al., (1997), J. Mol. Biol. 273:927-948; and Martin et al., (1989), Proc. Natl. Acad. Sci. USA 86:9268-9272. Public databases are also available to identify CDR sequences.

Preferably, the antibody or antigen binding fragment thereof comprises a HCVR/LCVR amino acid sequence pair wherein the HCVR has at least 80%, at least 85%, at least 90%, at least 95%, at least 98% or at least 99% sequence identity with a HCVR having an amino acid sequence of SEQ ID No. 1, or wherein the LCVR has at least 80%, at least 85%, at least 90%, at least 95%, at least 98% or at least 99% sequence identity with a LCVR having an amino acid sequence of SEQ ID No. 2. In some embodiments, the antibody or antigen binding fragment thereof comprises a HCVR/LCVR amino acid sequence pair wherein the HCVR has at least 80%, at least 85%, at least 90%, at least 95%, at least 98% or at least 99% sequence identity with a HCVR having an amino acid sequence of SEQ ID No. 1, and wherein the LCVR has at least 80%, at least 85%, at least 90%, at least 95%, at least 98% or at least 99% sequence identity with a LCVR having an amino acid sequence of SEQ ID No. 2.

Sequence identity of amino acid sequences is typically measured using sequence analysis software. Protein analysis software matches similar sequences using measures of similarity assigned to various substitutions, deletions and other modifications, such as conservative amino acid substitutions. For instance, GCG software contains programs such as GAP and BESTFIT which can be used with default parameters to determine sequence identity between different amino acid sequences (see, e.g., GCG Version 6.1). Amino acid sequences also can be compared using FASTA with default or recommended parameters, a program in GCG Version 6.1. FASTA (e.g., FASTA2 and FASTA3) provides alignments and percent sequence identity of the regions of the best overlap between the query and search sequences. Another algorithm is the computer program BLAST, especially BLASTP or TBLASTN, using default parameters (see e.g. Altschul et al. (1990) J. Mol. Biol. 215:403-410 and Altschul et al. (1997) Nucleic Acids Res. 25:3389-402, both of which are incorporated herein by reference).

An antibody or antigen binding fragment thereof for use in the present invention may comprise a HCVR comprising the amino acid sequence of SEQ ID No. 1 or a LCVR comprising the amino acid sequence SEQ ID No. 2. Preferably, an antibody or antigen binding fragment thereof for use in the invention comprises a HCVR/LCVR amino acid sequence pair of SEQ ID No. 1/ SEQ ID No. 2.

Antibodies for use in the invention may be selected from any class of immunoglobulins, including IgM, IgG, IgD, IgA, and IgE. Preferably, the antibody is an IgG antibody. Any isotype of IgG can be used, including IgG1, IgG2, IgG3, and IgG4. Variants of the IgG isotypes are also contemplated. The antibody may comprise sequences from more than one class or isotype.

An antibody for use in the invention may comprise a γ1, γ2, γ3, or γ4 human heavy chain constant region or a variant thereof. An antibody for use in the invention may comprises a lambda or a kappa human light chain constant region.

An antibody or antigen binding fragment thereof for use in the present invention may comprise a heavy chain amino acid sequence of SEQ ID No. 3 or a light chain amino acid sequence of SEQ ID No. 4. In some embodiments, the antibody or antigen binding fragment thereof may comprise a heavy chain amino acid sequence of SEQ ID No. 3 and a light chain amino acid sequence of SEQ ID No.4.

The antibody for use in the present invention may be tildrakizumab or an antigen binding fragment thereof. Antibodies (including antigen binding fragments thereof) are also envisaged for use in the invention which comprise the six CDRs of tildrakizumab; an HCVR/LCVR pair of tildrakizumab; or the heavy chain and light chain of tildrakizumab.

As was mentioned above, tildrakizumab is a high-affinity, humanised immunoglobulin (Ig) G1/κ monoclonal antibody specifically targeting the p19 subunit of IL-23 (Sinclair R et al., Expert Rev Clin Immunol. November 2018; 1-8), useful for the treatment of adults with moderate-to-severe plaque psoriasis who are candidates for systemic therapy (see the summary of product characteristics on Ilumetri, available from the European Medicines Agency (EMA)). Following completion of the phase III reSURFACE clinical trial programme, tildrakizumab was approval by the EMA in September 2018.

The term "antibody" as referred to herein includes whole antibodies as well as antigen binding fragments (i.e., "antigen-binding portion") or single chains thereof. As used herein, the term "antigen binding fragment thereof" and the like includes a reference to a fragment of an antibody that retains the ability to bind to and antagonise hIL-23. Examples of antibody fragments include Fab, Fab', F(ab')2, and Fv fragments; diabodies; linear antibodies; single-chain antibody molecules, e.g., sc-Fv; and multispecific antibodies formed from antibody fragments (see for example Holliger and Hudson, 2005, Nature Biotech. 23(9):1126-1136; Adair and Lawson, 2005, Drug Design Reviews - Online 2(3), 209-217). The methods for creating and manufacturing these antibody fragments are well known in the art (see for example Verma et al., 1998, Journal of Immunological Methods, 216, 165-181). Antibody fragments for use in the present invention include the fragments described in WO 2008/103432 in particular the antibody fragments of antibody h13B8b.

In general, the antibodies (and antigen-binding fragments thereof) for use in the present invention function by binding to and antagonizing human IL-23. Antibody affinities (e.g. for human IL-23) may be determined using standard analysis. In some embodiments, affinity may be measured by surface plasmon resonance, e.g. BIAcore or solution-affinity ELISA (e.g. at 25°C or 37°C). In one embodiment, affinity is measured by surface plasmon resonance (e.g. BIAcore SPR) at 25°C. K_{D} values of 77-129 pM and 69-116 pM have been determined by BIAcore for tildrakizumab in WO2008/103432 which is incorporated herein by reference (tildrakizumab is referred to as h13B8-b in WO2008/103432).

Preferred antibodies and antigen binding fragments are those which bind human IL-23 with an affinity constant (K_{D}) value of no more than about 1×10⁻⁷ M; preferably no more than about 1×10⁻⁸M; more preferably no more than about 1×10⁻⁹ M; and most preferably no more than about 1×10⁻¹⁰ M or even 1×10⁻¹¹ M. In certain embodiments, the antibodies or antigen-binding fragments for use in the invention bind human IL-23 with a K_{D} value less than about 50nM, less than about 25nM, less than about 10nM, less than about 5nM, less than about 2nM, less than about 1nM, less than about 800 pM, less than about 600 pM, less than about 400 pM, less than about 300 pM, less than about 200 pM, less than about 170 pM, less than about 150 pM, less than about 140 pM, less than about less than about 130 pM, less than about 120 pM, less than about 110 pM, less than about 100 pM, less than about 90 pM, less than about 80 pM, or less than about 70 pM.

These affinity constant (K_{D}) values may be applied to the antibody sequences described herein for use in the present invention. For example, an antibody or antigen-binding fragment thereof may comprise a set of six CDRs as defined herein and a K_{D} for hIL-23 as determined by SPR (Biacore) at 25°C of less than about 50nM, less than about 25nM, less than about 10nM, less than about 5nM, less than about 2nM, less than about 1nM, less than about 800 pM, less than about 600 pM, less than about 400 pM, less than about 300 pM, less than about 200 pM, less than about 170 pM, less than about 150 pM, less than about 140 pM, less than about less than about 130 pM, less than about 120 pM, less than about 110 pM, less than about 100 pM, less than about 90 pM, less than about 80 pM, or less than about 70 pM.

### Administration

Typically, the anti-IL-23 antibody as herein defined, such as tildrakizumab, for use in the invention is administered to the patient by injection. Preferably, the injection is a subcutaneous injection.

Preferably, the anti-IL-23 antibody as herein defined, such as tildrakizumab, is administered to the patient in an aqueous composition comprising L-histidine, L-histidine hydrochloride monohydrate, polysorbate 80, sucrose and the anti-IL23 antibody. A particularly preferred composition comprises L-Histidine, L-Histidine hydrochloride monohydrate, polysorbate 80, sucrose, 100 mg of the anti-IL23 antibody (e.g. tildrakizumab) and water for injection in a total volume of 1 mL.

Typically, the anti-IL-23 antibody as herein defined, such as tildrakizumab, is administered to the patient in an amount of 100 mg. However, in patients having certain characteristics (such as a high disease burden or a body weight of greater than 90 kg), the anti-IL-23 antibody as herein defined, such as tildrakizumab, is preferably administered to the patient in an amount of 200 mg.

The anti-IL-23 antibody as herein defined, such as tildrakizumab, may be administered at a frequency determined by a person skilled in the art. However, in a preferred embodiment, the anti-IL-23 antibody as herein defined, such as tildrakizumab, is administered to the patient once in week 0, once in week 4, and then once every subsequent twelfth week.

### Additional medications/separate treatment methods

The anti-IL-23 antibody may be administered together with an additional medication and/or the administration carried out in parallel with a separate treatment method for the treatment of plaque psoriasis or other types of psoriasis.

Such medications and/or treatments are well known to the skilled person, and include topical treatments (such as corticosteroids, coal tar, anthracene derivatives, vitamin A and D derivatives, and salicylic acid preparations), phototherapy (such as photochemotherapy (e.g., Psoralen-UVA), phototherapy (e.g., UVA, UVB, UVB311), blue light therapy, and tanning salon or home-administered UVB), conventional systemic anti-psioratic drugs different to fumarate-based drugs (such as systemic corticosteroids, cyclosporine, methotrexate, mycophenolate mofetil, azathioprine, leflunomide, tacrolimus and apremilast), and other treatments (such as tumour necrosis factor-alpha inhibitors, IL-17 inhibitors, IL-17R inhibitors, IL-12/23 p40 inhibitors, IL-23p19 inhibitors or experimental biological products, and other medications affecting the immune function e.g. cytostatics).

Alternatively, the anti-IL-23 antibody may be administered with additional medications or separate treatments for plaque psoriasis or other types of psoriasis.

### EXAMPLES

The following non-limiting Examples illustrate the invention.

### Example 1 - Treatment of plaque psoriasis using tildrakizumab (reSURFACE 1 and reSURFACE 2)

reSURFACE 1 was a 3-part phase III, double-blind, randomized controlled trial to compare the safety and efficacy of tildrakizumab with placebo in 772 adult patients with chronic plaque psoriasis over 64 weeks (Reich K et al., The Lancet. 2017;390(10091):276-288). Part I spanned from week 0 to week 16, part II spanned from week 16 to week 28, and part III spanned from week 28 to week 64. Patients in part I were randomly assigned (2:2: 1) at baseline to tildrakizumab 200 mg, tildrakizumab 100 mg, or placebo at baseline and week 4. In part II, patients received another dose at week 16 and those in placebo groups were re-randomized (1:1) to either tildrakizumab 200 mg or 100 mg. In part III, those who responded (PASI ≥75) and partial responders (PASI ≥50-<75) to tildrakizumab were re-randomized at week 28 to continue with the treatment or given a different dose of tildrakizumab or a placebo every 12 weeks until week 64.

The proportions of tildrakizumab groups (200 mg, 62%; 100 mg, 64%) achieving a PASI 75 response at week 12 were higher than in the placebo group (6%, p<0.0001). The frequencies of tildrakizumab groups (200 mg, 59%; 100 mg, 58%) achieving a PGA of "clear" or "minimal" at week 12 were higher than in the placebo group (7%, p<0.0001). Patients who initially received placebo and transitioned to tildrakizumab had a steady increase in clinical response until week 28, when the response plateaued to similar levels of patients taking tildrakizumab from baseline. The frequencies of the tildrakizumab groups (200 mg, 35% and 14% respectively; 100 mg, 35% and 14%, respectively) achieving PASI 90 and PASI 100 at week 12 were higher than in the placebo group (3% and 2% respectively, p<0.0001). The proportions of patients achieving a Dermatology Life Quality Index (DLQI) of 0 or 1 at week 12 in the tildrakizumab groups (200 mg, 44%; 100 mg, 42%) were higher than in the placebo group (5%, p<0.0001). In the tildrakizumab 200 mg group, 94% of responders and 40% of partial responders at week 28 maintained or achieved a PASI 75 response at week 64. In the tildrakizumab 100 mg group, 88% of responders and 75% of partial responders at week 28 maintained or achieved a PASI 75 response from weeks 28 to 64.

reSURFACE 2 was a 3-part phase III, double-blind, randomized controlled trial to compare the safety and efficacy of tildrakizumab with etanercept or placebo in 1090 adult patients with chronic plaque psoriasis over 52 weeks (Reich K et al., The Lancet. 2017;390(10091):276-288). Part I spanned from week 0 to week 16, part II spanned from week 16 to week 28, and part III spanned from week 28 to week 52. Patients were randomly assigned (2:2:1:2) at baseline to receive tildrakizumab 200 mg, tildrakizumab 100 mg, or placebo SC at baseline and week 4, or etanercept 50 mg twice weekly. In part II patients received another dose at week 16, both tildrakizumab groups received the same dose, whereas the placebo group was re-randomized (1:1) to either tildrakizumab 200 mg or tildrakizumab 100 mg. The etanercept group downgraded to once weekly. In part III, responders and partial responders to tildrakizumab at week 28 were randomized to continue the same treatment, switched to a different dose of tildrakizumab, or given a placebo every 12 weeks until week 52.

At week 12 of reSURFACE 2 trial: the proportions of patients achieving a PASI 75 response in the tildrakizumab groups (200 mg, 66%; 100 mg, 61%) were greater than in the placebo (6%, p<0.0001) and etanercept (48%, p=0.01) groups. The frequencies of tildrakizumab groups (200 mg, 59%; 100 mg, 55%) achieving "clear" or "minimal" PGA were greater than the placebo group (4%, p<0.0001). The frequency of PGA response in the tildrakizumab 200-mg group (59%) was higher compared with etanercept (48%, p=0.0031). The frequencies of achieving PASI 90 and PASI 100 were higher in both tildrakizumab groups (200 mg, 37% and 12%; 100 mg, 39% and 12%) than in placebo (1% and 0%, p<0.0001) and etanercept (21% and 5%, p<0.0014) groups. The proportions of patients achieving a DLQI of 0 or 1 were higher in the tildrakizumab groups (200 mg, 47%; 100 mg, 40%) than in the placebo group (8%, p<0.0001); 47% of patients in the tildrakizumab 200-mg group achieved a DLQI of 0 or 1 compared with 36% in the etanercept group (p=0.0029). In addition, in the tildrakizumab 200 mg group, 97% of responders and 67% of partial responders at week 28 maintained or achieved a PASI 75 response at week 52. In the tildrakizumab 100 mg group, 94% of responders and 68% of partial responders at week 28 maintained or achieved a PASI 75 response at week 52.

A number of the patients who were successfully treated with tildrakizumab during these clinical trials were suffering from FAE-resistant plaque psoriasis. That is to say, those patients had previously received unsuccessful treatments with DMF, but nevertheless were treated successfully with tildrakizumab. Thus, tildrakizumab was shown to provide an effective therapy for patients having fumaric acid ester-resistant plaque psoriasis

### Example 2 - Treatment of FAE-resistant plaque psoriasis using tildrakizumab-clinical trial

### Trial design

This is a multicentre, randomized, parallel group, open label phase IV clinical study in patients with moderate-to-severe chronic plaque psoriasis. The main aim of the study is to evaluate the efficacy, safety and tolerability of tildrakizumab in non-responder patients from DMF after 16 weeks of treatment. The study consists in two parts. Part 1 will include a Screening Period of four weeks (one visit) and the first 16 weeks of the Treatment Period (seven visits: three virtual visits and four on-centre visits). Part 2 will include the last 24 weeks of the Treatment Period (three visits) and a Safety Follow-up Period that includes one visit four weeks after the last study drug medication dose. In addition, patients who have received tildrakizumab will have another visit during the Safety Follow-up Period, 17 weeks after the last study drug medication dose. Thus, for most of patients receiving only DMF the study will include a total of 12 visits (three virtual visits and nine on-centre visits) during a period of 44 weeks. Likewise, for patients receiving DMF and tildrakizumab the study will include a total of 13 visits (three virtual visits and 10 on-centre visits) during a period of 57 weeks.

Overall, approximately 250 patients will be randomized across 33 study centres in Europe (approximately 7-8 patients per centre); in at least Germany, Netherlands and United Kingdom (UK). Adult male and female patients will be eligible to participate in the study if they are FAEs and biologic treatment-naive. Non-treatment-naive patients must follow the a wash-out period according to the current or last treatment for psoriasis.

In the Treatment Period, eligible patients will be randomized (1:1) to receive either DMF standard scheme or DMF optimised scheme from Baseline to Week 16. During study Part 1 the use of short courses of add-on mild or moderate potency topical corticosteroids (TCS) will be allowed from Baseline Visit up to Week 14. The use of TCS should have a clinical justification and the complete TCS treatment information must be documented. Patients achieving a PASI 75 response (responder patients) at Week 16 will discontinue the study and they will be treated at the judgment of the Investigator or according to the centre's routine clinical practice. Patients failing to achieve a PASI 75 response but achieving a PASI 50 response (partial responder patients) at Week 16 will continue with DMF treatment until Week 40. However, for patients continuing with DMF treatment and who do not achieve a clinically meaningful response at Week 24 at judgment of the Investigator, add-on mild or moderate potency TCS will be allowed from Week 24 to Week 40. The use of TCS should have a clinical justification and the complete TCS treatment information must be documented. Patients failing to achieve a PASI 50 response (non-responder patients) at Week 16 will be treated with tildrakizumab until Week 40. The primary efficacy endpoint of the study will be evaluated at Week 40 in these patients.

Tildrakizumab dose will be 100/200 mg administered as SC injection at Week 16, 20 and 32 as indicated by the SmPC. To improve tolerability, DMF dose will be taken orally and will be gradually increased up to a maximum of 720 mg/day. For the standardised scheme group, DMF dosing uptitration will follow the SmPC indication while for the optimised scheme group, uptitration will follow an optimised version of it.

Besides the Treatment Period, all patients will undergo a Screening Period and a Safety Follow-Up Period. The duration of the Treatment Period will vary among patients. Responder patients at Week 16 will participate in the study for approximately 24 weeks, partial patients will be participate for approximately 48 weeks, and non-responder patients will be participate for approximately 61 weeks.

The expected duration of the trial is 16 months.

### Rationale for trial design

All patients included in the study will start DMF treatment, although patients will be randomized to receive two different uptitration dosing schemes. Once all the patients have reached the DMF maximum tolerated daily dose, the treatment response will be assessed and patients with a poor treatment response will start treatment with tildrakizumab.

No type of control group has been included in the design of this study as this is an open phase IV clinical study where the therapeutic indication, posology and method of administration will follow the SmPCs of DMF and tildrakizumab.

### Rationale for trial population

In this phase IV clinical study, the patient population will be included following the therapeutic indications for which DMF and tildrakizumab have been approved for the EMA: adults with moderate-to-severe plaque psoriasis who are candidates for systemic therapy.

### Rationale for dose/s and dose regimes

In this phase IV clinical study, patients will be treated with DMF and tildrakizumab following the posology and method of administration in the SmPC of each study drug (subject to the parallel use of an optimised treatment protocol for DMF in some patients).

### Number of patients

The study will be conducted in approximately 33 study centres in Europe, in at least Germany, Netherlands and UK. It is anticipated that approximately 250 patients will be randomized into the study. Approximately 7-8 patients will be randomized per centre. Thus, 250 patients will be treated with DMF in the Part 1 of the study (125 patients in each DMF scheme), and assuming that 46% of these patients will be non-responders at Week 16 and a drop-out rate of 37% during the Part 1, it is anticipated that 72 patients will be treated with tildrakizumab in the Part 2 of the study.

### Inclusion criteria

Patients eligible for inclusion in this study have to fulfil all of the following criteria:
1. Ability to understand and comply with the requirements of the study and communicate with the Investigator, and written, signed and dated informed consent given before any study related activity is performed.
2. Male or female, aged 18 years at the time of the Screening Visit.
3. Diagnosed with chronic plaque psoriasis of at least 6 months prior to the Screening Visit, and has stable active plaque-type psoriasis (stable is defined as without clinically significant flares during the 12 weeks before randomization).
4. Moderate-to-severe plaque psoriasis at the Screening and Baseline visits as defined by PASI score of ≥ 10
5. Complete record of at least the last 12 months prior to the Screening Visit of psoriatic previous topical, phototherapy and non-biologic systemic treatments, if any.
6. Candidate for systemic treatment for the treatment of plaque psoriasis at the Screening Visit.
7. General good health, or a stable medical condition not considered likely to interfere with the conduct of the clinical study, as determined by the Investigator based upon results of medical history, laboratory results (within normal or clinically acceptable range limits) and physical examination (no clinical significant abnormal findings). Investigators are encouraged to consult with the Sponsor if there are questions regarding the significance of any out of range values.
8. Unlikely to conceive, as indicated by at least one "yes" answer to the following questions:
   a. Patient is a male.
   b. Patient is a surgically sterilized female by hysterectomy or bilateral tubal ligation.
   c. Patient is a postmenopausal female ≥45 years of age with >1 year since last menses. If a patient is <45 years of age, or cessation of menses is more than 3 months and less than 1 year, follicle stimulating hormone must be documented as elevated into the postmenopausal range (>60 mIU/mL) at the Screening visit.
   d. Patient is a non-sterilized and pre-menopausal female using a highly effective medically accepted method of contraception, during the study period and for 60 days after the last dose of the study drug. Highly effective methods of birth control are defined as a method with less than 1% failure rate (e.g. hormone implants, hormone injections, some intrauterine devices, vasectomized partner or sexual abstinence) or the use of two methods of contraception (e.g. one barrier method [condom, diaphragm or cervical/vault caps] with spermicide and one hormonal contraceptive [e.g. combined oral contraceptives, patch, vaginal ring, injectables and implants])
9. For female patients of child-bearing potential, a negative serum pregnancy test at the Screening Visit and a negative urine pregnancy test at the Baseline Visit. Additionally, they must agree to have urine pregnancy tests while on study medication.
10. Willingness of participating in the standardised photography procedure. The patients must be willing to give written informed consent.

### Identity of study drugs

### Dimethyl fumarate:

| | |
|---|---|
| Substance code/name: | Dimethyl fumarate, DMF (Skilarence^{®}) |
| Administration route: | Oral |
| Dose form: | Gastro-resistant tablet |
| Unit dose/strength: | Skilarence^{®} 30 mg and Skilarence^{®} 120 mg. |
| Qualitative formulation: | Lactose monohydrate, cellulose microcrystalline, croscarmellose sodium, colloidal anhydrous silica, magnesium stearate, talc, methacrylic acid-ethyl acrylate copolymer (1:1), triethyl citrate, titanium dioxide (E171), simethicone, indigo carmine (E132), sodium hydroxide |
| Frequency: | Daily and as per treatment scheme |
| Mode of administration: | Oral |
| Packaging Description: | Skilarence^{®} 30 mg: 42 gastro-resistant tablets in PVC/PVDC-aluminium blister packs |
| | Skilarence^{®} 120 mg: 180 gastro-resistant tablets in PVC/PVDC-aluminium blister packs |
| Manufacturer: | Industrias Farmacéuticas Almirall, S.A. Sant Feliu de Llobregat site |

Patients will begin DMF treatment with a low initial dose with subsequent gradual increases. Although two different uptitration schemes will be followed during the DMF treatment, at the end study Part 1 all patients will have reached the DMF maximum tolerated daily dose.

The DMF standardised scheme will follow the SmPC posology. As such, in the first week, patients will take DMF 30 mg once daily (one tablet in the evening). In the second week, patients will take DMF 30 mg twice daily (one tablet in the morning and one in the evening). In the third week, patients will take DMF 30 mg three times daily (one tablet in the morning, one at midday, and one in the evening). From the fourth week, treatment is switched to only one tablet of DMF 120 mg in the evening. Patients will then increase DMF dose by one 120 mg tablet per week at different times of day for the subsequent 5 weeks. Thus, in the fifth week, patients will take DMF 120 mg twice daily (one tablet in the morning and one in the evening). In the sixth week, patients will take DMF 120 mg three times daily (one tablet in the morning, one at midday, and one in the evening). In the seventh week, patients will take DMF 120 mg four times daily (one tablet in the morning, one at midday, and two in the evening). In the eighth week, patients will take DMF 120 mg five times daily (two tablets in the morning, one at midday, and two in the evening). In the ninth week, patients will take DMF 120 mg six times daily (two tablets in the morning, two at midday, and two in the evening) - the maximum daily dose. The maximum dosage will be maintained for the subsequent weeks.

In the optimised DMF treatment scheme, in the first week, patients will take DMF 60 mg daily (one DMF 30 mg tablet in the morning and one in the evening). In the second week, patients will take DMF 120 mg daily (two DMF 30 mg tablets in the morning and two in the evening). In the third week, patients will take DMF 180 mg daily (three DMF 30 mg tablets in the morning and three in the evening). From the fourth week onwards, treatment is switched to DMF 120 mg tablets. In the fourth week, patients will take DMF 240 mg daily (one DMF 120 mg tablet in the morning and one in the evening). In the fifth week, patients will take DMF 360 mg daily (one DMF 120 mg tablet in the morning and two in the evening). From the sixth to eighth week, patients will take DMF 480 mg daily (two DMF 120 mg tablets in the morning and two in the evening). If a PASI reduction of ≥ 30% is achieved at Week 8, no further increase of dosage is necessary. By contrast, if the PASI reduction is <30% at Week 8, uptitration will continue with DMF 600 mg daily (two DMF 120 mg tablets in the morning and three in the evening) in the ninth week and then DMF 720 mg daily (three DMF 120 mg tablets in the morning and three in the evening) from the tenth week onwards.

For both DMF treatment schemes, in case of significant individual intolerability to DMF at any time during the study, the dose should be reduced to the last tolerated dose. This dose should be maintained for at least seven days before a new dose increase is attempted.

If treatment success, defined as a PASI 75 response, is achieved before the maximum dose of 720 mg at Week 9 is reached in the standard scheme, no further increase of dose is necessary. Likewise, if treatment success is achieved before the maximum dose, 480 mg at Week 6 or 720 mg at Week 10, is reached in the optimised scheme, no further increase of dose is necessary. After clinically relevant improvement of the skin lesions has been achieved, Investigators should consider a gradual reduction of the daily dose of DMF to the maintenance dose required by the patient.

During the Treatment Period, gradual dose adjustments may be tailored on a patient by patient basis using the dosage strengths available in order to mitigate AEs.

All DMF modifications should be collected.

During the treatment with DMF, a complete blood count with differential should be performed every 3 months as is indicated in the SmPC. Action is needed when lymphocyte levels drop below 1.0 × 10⁹ cells/L, when leukocyte levels drop below 3.0 × 10⁹ cells/L, and when there is any clinically-relevant change in hepatic enzymes or in renal function and urine status.

In patients switching from DMF to tildrakizumab at week 16, blood tests will be performed to monitor leukocyte and lymphocyte counts.

### Tildrakizumab:

| | |
|---|---|
| Substance code/name: | Tildrakizumab (Ilumetri^{®}) |
| Administration route: | Subcutaneous injection |
| Dose form: | Solution for injection in pre-filled syringe |
| Unit dose/strength: | 100 mg |
| Qualitative formulation: | L-Histidine, L-Histidine hydrochloride monohydrate, polysorbate 80, sucrose, water for injections, 100 mg of tildrakizumab in 1 mL |
| Frequency: | At weeks 0, and 4 and every 12 weeks thereafter, corresponding to Week 16, Week 20 and Week 32 in this study |
| Mode of administration: | Subcutaneous injection |
| Packaging Description: | 1 mL solution in a type I glass pre-filled syringe with stainless steel 29G × ½" needle, covered with a needle shield and rigid needle shield of polypropylene with a fluropolymer lamination, plunger stopper assembled in a passive safety device |

In patients with certain characteristics (e.g. high disease burden, body weight ≥ 90 kg) 200 mg may provide greater efficacy. Investigators will be allowed to prescribe either of the two doses depending on the individual patient's characteristics at the beginning of the Part 2. Once a dose is chosen and treatment with tildrakizumab is initiated, dose changes (e.g. from 100mg to 200mg or vice versa) will not be allowed.

### Study treatment administration

DMF and tildrakizumab will be administrated according to the SmPC of each study drug.

DMF is for oral use and tablets must be swallowed whole with fluid during or immediately after a meal. The coating of the gastro-resistant tablets is designed to prevent gastric irritation. Therefore, the tablets should not be crushed, divided, dissolved or chewed. Regarding the daily dose, an uptitration will take place during the first weeks of the treatment in the Part 1 and two uptitration dosing schemes will be followed according to the treatment group.

Tildrakizumab is administered by SC injection. Injection sites should be alternated. Tildrakizumab should not be injected into areas where the skin is affected by plaque psoriasis or is tender, bruised, red, hard, thick, or scaly. The pre-filled syringe must not be shaken. Each pre-filled syringe is for single use only. The full amount of tildrakizumab should be injected according to the instructions for use provided in the package leaflet. While patients are in the trial, tildrakizumab will be administered by the Investigator or his/her designate during the visit.

### Scheduled activities and trial visits

The following sections indicate the timing of all study events.

### Screening period (week -4 to -1)

This period will start with the signature of the ICF, then will follow with Visit 1 (Screening Visit), and will end at the Visit 2 (Baseline Visit).

Prior to signature of the ICF, Investigators will evaluate eligibility of patients for entry in the trial by comparing past and current medical status, as documented in the patient's medical records, to the inclusion/exclusion criteria of the study (Section 10).

Eligible patients will receive a detailed description of all activities and requirements before signing the ICF to ensure their understanding and compliance with sample collection and clinical examinations.

Patients who require a washout period from prohibited concomitant treatments should be seen and sign the ICF prior to the Screening Visit, to ensure the necessary washout before.

No trial assessments will be performed on that date and the Screening Visit will be scheduled according to the washout length required for the specific medication stopped.

### VISIT 1:

At Visit 1, the following activities and assessments will be performed within 28 days prior to Visit 2:
- Patient's ICF signature
- Review of medical and surgical history and current diseases.
- Documentation of prior and concomitant medication/non-drug therapies
- Measurement of height, body weight, waist circumference and waist-hip ratio
- Complete physical examination
- Documentation of demographic and habits information
- Measurement of vital signs
- Safety laboratory examinations (haematology, clinical chemistry and urinalysis)
- Serum pregnancy test
- BSA assessment
- PASI assessment
- Determination of patients eligibility (inclusion and exclusion criteria)
- AEs

### Treatment period

Activities and assessments during Part 1 and Part 2 of Treatment Period are detailed below. Day 1 is considered to be the first day of study drug administration. Window period for each visit is ± 3 days.

Visit 8 (Week 16) belongs to Part 1 Treatment Period, however, the study drug administration at this visit is considered to be the first study drug administration of the Part 2 Treatment Period.

Only in patients with palmar/plantar or scalp involvement the Palmoplantar and Scalp PGA assessments will be performed, respectively.

### VISIT 2 OR BASELINE VISIT (WEEK 0, DAY 1)

At Visit 2, the following activities and assessments will be performed:
- Re-confirmation of patient's eligibility, including laboratory assessments review
- Report changes to concomitant medication
- BSA assessment
- PASI assessment
- PGA, Palmoplantar PGA and Scalp PGA
- Pruritus-VAS electronic assessment
- MOS Sleep electronic assessment
- DLQI electronic assessment
- Skindex-16 electronic assessment
- Electronic diary (patient instructions for correct completing of the diary)
- Photographs of the selected treatment area (if applicable)
- Safety laboratory examinations (haematology, clinical chemistry and urinalysis)
- Urine pregnancy test
- Randomization
- Dispensing of the DMF, including patient instructions on the number of tables and the timings taking the DMF medication
- Administration of the first DMF dose, for patients randomized to optimised scheme, during the visit and administration of the DMF dose outside the visit
- Reporting new or on-going AEs

### VISIT 3 (WEEK 1), VISIT 4 (WEEK 4) AND VISIT 5 (WEEK 6)

Visits 3, 4 and 5 are virtual visits and the following activities and assessments will be performed:
- Report changes to concomitant medication
- Administration of the DMF dose outside the visit
- Electronic diary (check the diary)
- Study App check
- Reporting new or on-going AEs

### VISIT 6 (WEEK 8)

At Visit 6, the following activities and assessments will be performed:
- Report changes to concomitant medication
- BSA assessment
- PASI assessment
- PGA, Palmoplantar PGA and Scalp PGA
- Pruritus-VAS electronic assessment
- DLQI electronic assessment
- Skindex-16 electronic assessment
- Electronic diary (check the diary)
- Administration of the DMF dose outside the visit
- Reporting new or on-going AEs

### VISIT 7 (WEEK 12)

At Visit 7, the following activities and assessments will be performed:
- Report changes to concomitant medication
- Safety laboratory examinations (haematology, clinical chemistry and urinalysis)
- Urine pregnancy test
- Electronic diary (check the diary)
- Administration of the DMF dose outside the visit
- Reporting new or on-going AEs

### VISIT 8 (WEEK 16)

At Visit 8, the following activities and assessments will be performed:
- Brief physical examination
- Measurement of body weight, waist circumference and waist-hip ratio
- Report changes to concomitant medication
- Measurement of vital signs
- BSA assessment
- PASI assessment
- PGA, Palmoplantar PGA and Scalp PGA
- Pruritus-VAS electronic assessment
- MOS Sleep electronic assessment
- DLQI electronic assessment
- Skindex-16 electronic assessment
- Electronic diary (check the diary)
- Photographs of the selected treatment area (if applicable)
- Safety laboratory examinations (haematology, clinical chemistry and urinalysis)
- Urine pregnancy test
- Treatment allocation
- Dispensing and returning of DMF
- Administration of the first tildrakizumab dose, for patients allocated in tildrakizumab treatment, during the visit and administration of the DMF dose outside the visit for patients continuing in DMF treatment.
- Reporting new or on-going AEs

### VISIT 9 (WEEK 20 OR WEEK 24)

At Visit 9 will be on Week 20 for patients in tildrakizumab treatment or on Week 24 in patients in DMF treatment. The following activities and assessments will be performed:
- Report changes to concomitant medication
- BSA assessment
- PASI assessment
- PGA, Palmoplantar PGA and Scalp PGA
- Pruritus-VAS electronic assessment
- Electronic diary (check the diary)
- Safety laboratory examinations (haematology, clinical chemistry and urinalysis)
- Urine pregnancy test
- Dispensing and returning of DMF
- Administration of the second tildrakizumab dose, for patients allocated in tildrakizumab treatment, during the visit and administration of the DMF dose outside the visit for patients continuing in DMF treatment.
- Reporting new or on-going AEs

### VISIT 10 (WEEK 32)

At Visit 10, the following activities and assessments will be performed:
- Report changes to concomitant medication
- BSA assessment
- PASI assessment
- PGA, Palmoplantar PGA and Scalp PGA
- Pruritus-VAS electronic assessment
- DLQI electronic assessment
- Skindex-16 electronic assessment
- Electronic diary (check the diary)
- Safety laboratory examinations (haematology, clinical chemistry and urinalysis)
- Urine pregnancy test
- Dispensing and returning of DMF
- Administration of the third tildrakizumab dose, for patients allocated in tildrakizumab treatment, during the visit and administration of the DMF dose outside the visit for patients continuing in DMF treatment
- Reporting new or on-going AEs

### VISIT 11 (WEEK 40)

At Visit 11, the following activities and assessments will be performed:
- Brief physical examination
- Measurement of body weight, waist circumference and waist-hip ratio
- Report changes to concomitant medication
- Measurement of vital signs
- BSA assessment
- PASI assessment
- PGA, Palmoplantar PGA and Scalp PGA
- Pruritus-VAS electronic assessment
- MOS Sleep electronic assessment
- DLQI electronic assessment
- Skindex-16 electronic assessment
- Electronic diary (check the diary)
- Photographs of the selected treatment area (if applicable)
- Safety laboratory examinations (haematology, clinical chemistry and urinalysis)
- Urine pregnancy test
- Administration of the DMF dose outside the visit for patients continuing in DMF treatment
- Returning of DMF
- Reporting new or on-going AEs

### Safety Follow-up period

A Safety Follow-Up Period will begin after Visit 11 (Week 40) for completed patients, Visit 8 for responder patients at Week 16 and the premature discontinuation visit (when applicable), in order to check if any new AEs have occurred and to do follow-up of any previous AEs that was ongoing. This information will be recorded.

### VISIT 12

Visit 12 will be performed in all patients, 4 weeks after the last dose administration of the study drugs, and the following activity will be performed:
- Reporting new or on-going AEs

### VISIT 13

Visit 13 will be performed in patients receiving tildrakizumab, 17 weeks after the last dose administration of the study drug, and the following activity will be performed:
- Reporting new or on-going AEs

### UNSCHEDULED VISIT

An unscheduled visit will be performed in female patients with a positive urine pregnancy test in order to perform a serum pregnancy test.

### Endpoints

The following outcomes of interest will be measured:
- Proportion of patients who are unresponsive to treatment with DMF achieving a PASI 75 score at week 40 following treatment with tildrakizumab.
- Proportion of patients achieving PASI 50, PASI 75, PASI 90, and PASI 100 responses at each visit of the Part 2.
- Proportion of patients achieving absolute PASI scores of <5, <3 and <1 at each visit of the Part 2
- Absolute PASI score and absolute and percentage change in the absolute PASI score at each visit of the Part 2
- Absolute BSA score and change in the absolute BSA score at each visit of the Part 2.
- Proportion of patients achieving PGA, ScPGA and PPPGA scores of 0 or 1 at each visit of the Part 2
- Absolute PGA, ScPGA and PPPGA scores and absolute and percentage change in the absolute PGA, ScPGA and PPPGA scores at each visit of the Part 2.
- Proportion of patients achieving DLQI score of 0 or 1 at Week 32 and Week 40 Visits of the Part 2.
- Absolute DLQI score and absolute and percentage change in the absolute DLQI score at Week 32 and Week 40 Visits of the Part 2.
- Absolute Skindex-16 score and absolute and percentage change in the absolute Skindex-16 score at Week 32 and Week 40 Visits of the Part 2.
- Absolute pruritus-VAS score and absolute and percentage change in the absolute pruritus-VAS score at each visit of the Part 2.
- Absolute MOS score and absolute and percentage change in the absolute MOS score at Week 40 Visit of the Part 2.

### Statistics

Approximately 250 patients will be enrolled in this trial to be treated DMF during 16 weeks. Assuming that 46% of patients treated with DMF will be non-responders at Week 16 and considering a drop-out rate of 37% during the first 16 weeks of the trial, it is predicted that there will be 72 non-responder patients still in treatment at Week 16.

### Conclusions

Approximately 250 patients will be enrolled in this trial to be treated DMF during 16 weeks. Assuming that 46% of patients treated with DMF will be non-responders at Week 16 and considering a drop-out rate of 37% during the first 16 weeks of the trial, there will be 72 non-responder patients still in treatment at Week 16.

A total sample size of 72 non-responder patients at Week 16 will allow the estimation of around 57% of patients treated with tildrakizumab achieving a PASI 75 response at Week 40, with a precision of 11.5% with a 95% Confidence Interval (CI); i.e. a 95% CI for the proportion of patients with a PASI 75 response at week 40 of 45.5% - 68.5%.

Thus, tildrakizumab provides an effective therapy for those patients having fumaric acid ester-resistant plaque psoriasis.

### Brief Description of the Sequence Listing

SEQ ID NO: 1 provides the sequence of the HCVR.
SEQ ID NO: 2 provides the sequence of the LCVR.
SEQ ID NO: 3 provides the sequence of the heavy chain.
SEQ ID NO: 4 provides the sequence of the light chain.
SEQ ID NO: 5 provides the sequence of the HCDR1.
SEQ ID NO: 6 provides the sequence of the HCDR2.
SEQ ID NO: 7 provides the sequence of the HCDR3.
SEQ ID NO: 8 provides the sequence of the LCDR1.
SEQ ID NO: 9 provides the sequence of the LCDR2.
SEQ ID NO: 10 provides the sequence of the LCDR3.

### Sequence listing

SEQ ID NO: 1 (HCVR) 

1               5                   10                  15


            20                  25                  30


        35                  40                  45


    50                  55                  60


 
65                  70                  75                  80


                85                  90                  95


            100                 105                 110


        115
SEQ ID NO: 2 (LCVR) 
Asp Ile Gln Met Thr Gln Ser Pro Ser Ser Leu Ser Ala Ser Val Gly
1               5                   10                  15

Asp Arg Val Thr Ile Thr Cys Arg Thr Ser Glu Asn Ile Tyr Ser Tyr
            20                  25                  30

Leu Ala Trp Tyr Gln Gln Lys Pro Gly Lys Ala Pro Lys Leu Leu Ile
        35                  40                  45

Tyr Asn Ala Lys Thr Leu Ala Glu Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln His His Tyr Gly Ile Pro Phe
                85                  90                  95

Thr Phe Gly Gln Gly Thr Lys Val Glu Ile Lys Arg
            100                 105
SEQ ID NO: 3 (heavy chain) 
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Ile Phe Ile Thr Tyr
            20                  25                  30

Trp Met Thr Trp Val Arg Gln Ala Pro Gly Gln Gly Leu Glu Trp Met
        35                  40                  45

Gly Gln Ile Phe Pro Ala Ser Gly Ser Ala Asp Tyr Asn Glu Lys Phe
    50                  55                  60

Glu Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Gly Gly Gly Gly Phe Ala Tyr Trp Gly Gln Gly Thr Leu Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
 
195                 200                 205

Lys Val Asp Lys Lys Val Glu Pro Lys Ser Cys Asp Lys Thr His Thr
    210                 215                 220

Cys Pro Pro Cys Pro Ala Pro Glu Leu Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Asp Glu Leu Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400
 
Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445
SEQ ID NO: 4 (light chain) 
Asp Ile Gln Met Thr Gln Ser Pro Ser Ser Leu Ser Ala Ser Val Gly
1               5                   10                  15

Asp Arg Val Thr Ile Thr Cys Arg Thr Ser Glu Asn Ile Tyr Ser Tyr
            20                  25                  30

Leu Ala Trp Tyr Gln Gln Lys Pro Gly Lys Ala Pro Lys Leu Leu Ile
        35                  40                  45

Tyr Asn Ala Lys Thr Leu Ala Glu Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln His His Tyr Gly Ile Pro Phe
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125
 
Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210
SEQ ID NO: 5 (HCDR1) 
Gly Tyr Ile Phe Ile Thr Tyr Trp Met Thr
1               5                   10
SEQ ID NO: 6 (HCDR2) 
Gln Ile Phe Pro Ala Ser Gly Ser Ala Asp Tyr Asn Glu Lys Phe Glu
1               5                   10                  15

Gly
SEQ ID NO: 7 (HCDR3) 
Gly Gly Gly Gly Phe Ala Tyr
1               5
SEQ ID NO: 8 (LCDR1) 
Arg Thr Ser Glu Asn Ile Tyr Ser Tyr Leu Ala
1               5                   10
SEQ ID NO: 9 (LCDR2) 
Asn Ala Lys Thr Leu Ala Glu
1               5
SEQ ID NO: 10 (LCDR3)

Gln His His Tyr Gly Ile Pro Phe Thr
                         1               5

## Claims

1. An anti-IL23 antibody for use in a method of treating fumaric acid ester-resistant plaque psoriasis in a patient, said method comprising administering a therapeutically-effective amount of the anti-IL23 antibody to said patient, wherein said anti-IL23 antibody comprises a set of six CDRs, HCDR1, HCDR2, HCDR3, LCDR1, LCDR2 and LCDR3, wherein HCDR1 has the amino acid sequence of SEQ ID NO: 5; HCDR2 has the amino acid sequence of SEQ ID NO: 6; HCDR3 has the amino acid sequence of SEQ ID NO: 7; LCDR1 has the amino acid sequence of SEQ ID NO: 8; LCDR2 has the amino acid of SEQ ID NO: 9; and LCDR3 has the amino acid sequence of SEQ ID NO: 10.

2. The anti-IL23 antibody for use according to claim 1, wherein the anti-IL23 antibody:
(i) comprises a HCVR/LCVR amino acid sequence pair wherein the HCVR has at least 80%, at least 85%, at least 90%, at least 95%, at least 98% or at least 99% sequence identity with a HCVR having an amino acid sequence of SEQ ID No. 1, and the LCVR has at least 80%, at least 85%, at least 90%, at least 95%, at least 98% or at least 99% sequence identity with a LCVR having an amino acid sequence of SEQ ID No. 2; or
(ii) comprises a HCVR/LCVR amino acid sequence pair of SEQ ID No. 1/ SEQ ID No. 2.

3. The anti-IL23 antibody for use according to claim 1 or 2, wherein: (i) the anti-IL23 antibody comprises a heavy chain amino acid sequence of SEQ ID No. 3 and a light chain amino acid sequence of SEQ ID No.4; or (ii) the anti-IL23 antibody is tildrakizumab.

4. The anti-IL23 antibody for use according to any one of the preceding claims, wherein the anti-IL23 antibody is an antigen-binding fragment, and wherein the antigen-binding fragment is optionally selected from the group consisting of: Fab, Fab', F(ab')2, Fv, a diabody, and a sc-Fv.

5. The anti-IL23 antibody for use according to any one of the preceding claims, wherein the fumaric acid ester-resistant plaque psoriasis is a moderate to severe plaque psoriasis, and is preferably moderate plaque psoriasis or severe plaque psoriasis.

6. The anti-IL23 antibody for use according to any one of the preceding claims, wherein the fumaric acid ester is dimethyl fumarate.

7. The anti-IL23 antibody for use according to claim 6, wherein the dimethyl fumarate-resistant plaque psoriasis is one in which the patient exhibits a reduction of <50% in PASI score compared to the baseline score following a DMF treatment regime lasting for 16 weeks and involving DMF 30 mg once daily in the first week, DMF 30 mg twice daily in the second week, DMF 30 mg three times daily in the third week, DMF 120 mg once daily in the fourth week, DMF 120 mg twice daily in the fifth week, DMF 120 mg three times daily in the sixth week, DMF 120 mg four times daily in the seventh week, DMF 120 mg five times daily in the eighth week, and DMF 120 mg six times daily in the ninth and subsequent weeks.

8. The anti-IL23 antibody for use according to any one of the preceding claims, wherein the anti-IL23 antibody is administered to the patient by injection, preferably by subcutaneous injection.

9. The anti-IL23 antibody for use according to any one of the preceding claims, wherein the anti-IL23 antibody is administered to the patient in an aqueous composition comprising L-histidine, L-histidine hydrochloride monohydrate, polysorbate 80, sucrose and the anti-IL23 antibody.

10. The anti-IL23 antibody for use according to any one of the preceding claims, wherein the anti-IL23 antibody is administered to the patient in an amount of either 100 mg or 200 mg.

11. The anti-IL23 antibody for use according to any one of the preceding claims, wherein the anti-IL23 antibody is administered to the patient once in week 0, once in week 4, and then once every subsequent twelfth week.

12. The anti-IL23 antibody for use according to any one of claims 1 to 11, wherein the patient is a human and is preferably an adult.

13. The anti-IL23 antibody for use according to any one of claims 1 to 12, wherein the method further comprises administering an additional medication and/or carrying out a separate parallel treatment method for the treatment of plaque psoriasis or other types of psoriasis.

## Patentansprüche

1. Anti-IL23-Antikörper für die Verwendung in einem Verfahren zum Behandeln von Fumarsäureester-resistenter Plaque-Psoriasis bei einem Patienten, wobei das Verfahren das Verabreichen einer therapeutisch wirksamen Menge des Anti-IL23-Antikörpers an den Patienten umfasst, wobei der Anti-IL23-Antikörper einen Satz von sechs CDRs, HCDR1, HCDR2, HCDR3, LCDR1, LCDR2 und LCDR3 umfasst, wobei HCDR1 die Aminosäuresequenz von SEQ ID NO: 5 aufweist; HCDR2 die Aminosäuresequenz von SEQ ID NO: 6 aufweist; HCDR3 die Aminosäuresequenz von SEQ ID NO: 7 aufweist; LCDR1 die Aminosäuresequenz von SEQ ID NO: 8 aufweist; LCDR2 die Aminosäuresequenz von SEQ ID NO: 9 aufweist; und LCDR3 die Aminosäuresequenz von SEQ ID NO: 10 aufweist.

2. Anti-IL23-Antikörper für die Verwendung nach Anspruch 1, wobei der Anti-IL23-Antikörper:
(i) ein HCVR/LCVR-Aminosäuresequenzpaar umfasst, wobei der HCVR wenigstens 80 %, wenigstens 85 %, wenigstens 90 %, wenigstens 95 %, wenigstens 98 % oder wenigstens 99 % Sequenzidentität mit einer HCVR mit einer Aminosäuresequenz von SEQ ID NO: 1 aufweist, und die LCVR wenigstens 80 %, wenigstens 85 %, wenigstens 90 %, wenigstens 95 %, wenigstens 98 % oder wenigstens 99 % Sequenzidentität mit einer LCVR mit einer Aminosäuresequenz der SEQ ID NO: 2 aufweist; oder
(ii) ein HCVR/LCVR-Aminosäuresequenzpaar der SEQ ID NO: 1 / SEQ ID NO:2 umfasst.

3. Anti-IL23-Antikörper für die Verwendung nach Anspruch 1 oder 2, wobei: (i) der Anti-IL23-Antikörper eine Aminosäuresequenz der schweren Kette von SEQ ID NO: 3 und eine Aminosäuresequenz der leichten Kette von SEQ ID NO: 4 umfasst; oder (ii) der Anti-IL23-Antikörper Tildrakizumab ist.

4. Anti-IL23-Antikörper für die Verwendung nach einem der vorhergehenden Ansprüche, wobei der Anti-IL23-Antikörper ein antigenbindendes Fragment ist, und wobei das antigenbindende Fragment optional aus der Gruppe ausgewählt ist, bestehend aus: Fab, Fab', F(ab')2, Fv, einem Diabody und einem sc-Fv.

5. Anti-IL23-Antikörper für die Verwendung nach einem der vorhergehenden Ansprüche, wobei die Fumarsäureester-resistente Plaque-Psoriasis eine mittelschwere bis schwere Plaque-Psoriasis ist, und vorzugsweise eine mittelschwere Plaque-Psoriasis oder schwere Plaque-Psoriasis ist.

6. Anti-IL23-Antikörper für die Verwendung nach einem der vorhergehenden Ansprüche, wobei der Fumarsäureester Dimethylfumarat ist.

7. Anti-IL23-Antikörper für die Verwendung nach Anspruch 6, wobei die Dimethylfumarat-resistente Plaque-Psoriasis eine ist, bei der der Patient eine Reduktion der PASI-Bewertungsziffer von <50 % im Vergleich zu der Ausgangsbewertungsziffer nach einem DMF-Behandlungsregime zeigt, das 16 Wochen andauert und DMF 30 mg einmal täglich in der ersten Woche, DMF 30 mg zweimal täglich in der zweiten Woche, DMF 30 mg dreimal täglich in der dritten Woche, DMF 120 mg einmal täglich in der vierten Woche, DMF 120 mg zweimal täglich in der fünften Woche, DMF 120 mg dreimal täglich in der sechsten Woche, DMF 120 mg viermal täglich in der siebten Woche, DMF 120 mg fünfmal täglich in der achten Woche und DMF 120 mg sechsmal täglich in der neunten und den folgenden Wochen einschließt.

8. Anti-IL23-Antikörper für die Verwendung nach einem der vorhergehenden Ansprüche, wobei der Anti-IL23-Antikörper dem Patienten durch Injektion, vorzugsweise durch subkutane Injektion, verabreicht wird.

9. Anti-IL23-Antikörper für die Verwendung nach einem der vorhergehenden Ansprüche, wobei der Anti-IL23-Antikörper dem Patienten in einer wässrigen Zusammensetzung verabreicht wird, die L-Histidin, L-Histidinhydrochloridmonohydrat, Polysorbat 80, Saccharose und den Anti-IL23-Antikörper umfasst.

10. Anti-IL23-Antikörper für die Verwendung nach einem der vorhergehenden Ansprüche, wobei der Anti-IL23-Antikörper dem Patienten in einer Menge von entweder 100 mg oder 200 mg verabreicht wird.

11. Anti-IL23-Antikörper für die Verwendung nach einem der vorhergehenden Ansprüche, wobei der Anti-IL23-Antikörper dem Patienten einmal in Woche 0, einmal in Woche 4 und dann einmal in jeder nachfolgenden zwölften Woche verabreicht wird.

12. Anti-IL23-Antikörper für die Verwendung nach einem der Ansprüche 1 bis 11, wobei der Patient menschlich ist und vorzugsweise ein Erwachsener ist.

13. Anti-IL23-Antikörper für die Verwendung nach einem der Ansprüche 1 bis 12, wobei das Verfahren ferner das Verabreichen einer zusätzlichen Medikation und/oder das Ausführen eines separaten parallelen Behandlungsverfahrens für die Behandlung von Plaque-Psoriasis oder andere Arten von Psoriasis umfasst.

## Revendications

1. Anticorps anti-IL23 destiné à être utilisé dans un procédé de traitement du psoriasis en plaques résistant à l'ester d'acide fumarique chez un patient, ladite méthode comprenant l'administration d'une quantité thérapeutiquement efficace de l'anticorps anti-IL23 audit patient, ledit anticorps anti-IL23 comprenant un ensemble de six CDR, HCDR1, HCDR2, HCDR3, LCDR1, LCDR2 et LCDR3, dans lequel HCDR1 a la séquence d'acides aminés de SEQ ID NO : 5 ; HCDR2 a la séquence d'acides aminés de SEQ ID NO : 6 ; HCDR3 a la séquence d'acides aminés de SEQ ID NO : 7 ; LCDR1 a la séquence d'acides aminés de SEQ ID NO : 8 ; LCDR2 a l'acide aminé de SEQ ID NO : 9 ; et LCDR3 a la séquence d'acides aminés de SEQ ID NO : 10.

2. Anticorps anti-IL23 destiné à être utilisé selon la revendication 1, dans lequel l'anticorps anti-IL23 :
(i) comprend une paire de séquences d'acides aminés HCVR/LCVR dans lequel la HCVR présente au moins 80 %, au moins 85 %, au moins 90 %, au moins 95 %, au moins 98 % ou au moins 99 % d'identité de séquence avec une HCVR présentant une séquence d'acides aminés de SEQ ID No. 1, et le LCVR présente au moins 80 %, au moins 85 %, au moins 90 %, au moins 95 %, au moins 98 % ou au moins 99 % d'identité de séquence avec une LCVR présentant une séquence d'acides aminés de SEQ ID No. 2 ; ou
(ii) comprend une paire de séquences d'acides aminés HCVR/LCVR de SEQ ID No. 1/SEQ ID No. 2.

3. Anticorps anti-IL23 destiné à être utilisé selon la revendication 1 ou 2, dans lequel :
(i) l'anticorps anti-IL23 comprend une séquence d'acides aminés de chaîne lourde de SEQ ID No. 3 et une séquence d'acides aminés de chaîne légère de SEQ ID No. 4 ; ou
(ii) l'anticorps anti-IL23 est le tildrakizumab.

4. Anticorps anti-IL23 destiné à être utilisé selon l'une quelconque des revendications précédentes, dans lequel l'anticorps anti-IL23 est un fragment de liaison à l'antigène, et dans lequel le fragment de liaison à l'antigène est éventuellement choisi dans le groupe constitué de : Fab, Fab', F(ab')2, Fv, un dianticorps et un sc-Fv.

5. Anticorps anti-IL23 destiné à être utilisé selon l'une quelconque des revendications précédentes, dans lequel le psoriasis en plaques résistant à l'ester d'acide fumarique est un psoriasis en plaques modéré à sévère, et est de préférence un psoriasis en plaques modéré ou un psoriasis en plaques sévère.

6. Anticorps anti-IL23 destiné à être utilisé selon l'une quelconque des revendications précédentes, dans lequel l'ester d'acide fumarique est le fumarate de diméthyle.

7. Anticorps anti-IL23 destiné à être utilisé selon la revendication 6, dans lequel le psoriasis en plaques résistant au diméthyl fumarate est un psoriasis dans lequel le patient présente une réduction de < 50 % du score PASI par rapport au score de base après un régime de traitement au DMF d'une durée de 16 semaines et impliquant 30 mg de DMF une fois par jour pendant la première semaine, 30 mg de DMF deux fois par jour pendant la deuxième semaine, 30 mg de DMF trois fois par jour pendant la troisième semaine, 120 mg de DMF une fois par jour pendant la quatrième semaine, 120 mg de DMF deux fois par jour pendant la cinquième semaine, 120 mg de DMF trois fois par jour pendant la sixième semaine, 120 mg de DMF quatre fois par jour pendant la septième semaine, 120 mg de DMF cinq fois par jour pendant la huitième semaine et 120 mg de DMF six fois par jour pendant la neuvième semaine et les semaines suivantes.

8. Anticorps anti-IL23 destiné à être utilisé selon l'une quelconque des revendications précédentes, dans lequel l'anticorps anti-IL23 est administré au patient par injection, de préférence par injection sous-cutanée.

9. Anticorps anti-IL23 destiné à être utilisé selon l'une quelconque des revendications précédentes, dans lequel l'anticorps anti-IL23 est administré au patient dans une composition aqueuse comprenant de la L-histidine, du chlorhydrate de L-histidine monohydraté, du polysorbate 80, du saccharose et l'anticorps anti-IL23.

10. Anticorps anti-IL23 destiné à être utilisé selon l'une quelconque des revendications précédentes, dans lequel l'anticorps anti-IL23 est administré au patient en une quantité de 100 mg ou de 200 mg.

11. Anticorps anti-IL23 destiné à être utilisé selon l'une quelconque des revendications précédentes, dans lequel l'anticorps anti-IL23 est administré au patient une fois dans la semaine 0, une fois dans la semaine 4, puis une fois toutes les douzièmes semaines suivantes.

12. Anticorps anti-IL23 destiné à être utilisé selon l'une quelconque des revendications 1 à 11, dans lequel le patient est un humain et est de préférence un adulte.

13. Anticorps anti-IL23 destiné à être utilisé selon l'une quelconque des revendications 1 à 12, dans lequel le procédé comprend en outre l'administration d'un médicament supplémentaire et/ou la mise en œuvre d'un procédé de traitement parallèle distinct pour le traitement du psoriasis en plaques ou d'autres types de psoriasis.
